# EUROPEAN PATENT APPLICATION

(11) **EP 4 083 204 A1**
(43) Date of publication of application: **02.11.2022**
(21) Application number: 21171640.2
(22) Date of filing: 30.04.2021
(51) Int. Cl.: C12N 15/10, C12N 15/67

(54) **GENE WIDE RANDOMIZATION**

(71) Applicant: Wageningen Universiteit, 6708 PB Wageningen (NL)
(72) Inventor: NIEUWKOOP, Thijs, 6515 XS Nijmegen (NL); VAN DER OOST, John, 6871HZ Renkum (NL); CLAASSENS, Nicolaas Joannes Hubertus Petrus, 6708 LR Wageningen (NL)
(74) Representative: V.O.

(57) **Abstract**

The invention relates to methods of generating codon-randomized libraries for expressing a protein of interest in a host cell.

## Description

FIELD: The invention relates to methods for optimizing a sequence such as a coding sequence for functional expression of a heterologous protein in a host cell. The presented unbiased approach for optimization of a sequence relies on the provision of an expression library of synonymous coding sequences for a protein of interest. Expression of this library in a given host cell allows for selection of a host cell-specific optimized coding sequence.

### 1. BACKGROUND TO THE INVENTION

Production of proteins is a frequent activity in academic as well as industrial laboratories. However, the success rate of functional protein production varies substantially from case to case because there are many variables which potentially influence gene expression (Welch et al., 2009. PLoS ONE 4: e7002; Parret et al., 2016. Current Opinion Struct Biol 38: 155-162). Some of these variables can be easily manipulated by, for example, choice of a suitable expression vector, including a suitable promoter, and removal of premature start codons.

A consequence of degeneracy in the genetic code, with 61 different codons (triplets of nucleotides) encoding 20 amino acid residues, is that all amino acid residues, except for methionine and tryptophan, are encoded by more than one codon. During translation of a coding messenger RNA sequence by ribosomes, these codons are recognised by complementary transfer RNA (tRNA) molecules that deliver specific amino acids to the ribosome.

It is known that different codons encoding the same amino acid (synonymous codons) typically do not occur with equal frequency in organisms and their nonuniform occurrence is known as 'codon usage bias' or 'codon bias' (Grantham et al., 1980. Nucleic Acids Res 8: R49-R62). Codon bias relates to the fact that some codons are well recognised by a corresponding tRNA and result in relatively fast translation of a protein, while other codons result in less efficient translation.

In many organisms such as *Escherichia coli* and *Saccharomyces cerevisiae,* translational selection has been demonstrated, with positive correlations existing between codon bias of genes and corresponding protein levels (Lithwick and Margalit, 2003. Genome Res. 13: 2665-2673; Ghaemmaghami et al., 2003. Nature 425: 737-741). This means that heterologous expression may require adaptation of the codon bias to adjust it to match with the particular protein synthesis machinery of a production organism.

This knowledge has been underscored, resulting in the provision of algorithms to predict putative non-optimal codon usage (Zhang et al., 2009. Nat Struct Mol Biol 16: 274-280) or to optimize codon usage (US Patent 8,326,547), methods for randomly introducing codon-mutations in a target nucleic acid molecule (e.g. WO2014201416) and methods employing computational analysis for reassembling codon-randomized nucleic acid fragments (e.g. EP2921560).

However, other factors such as GC content, RNA stability, and secondary structure of the resulting mRNA may influence the level of protein expression in an organism. Consequently, algorithms that are aimed at codon optimization as sole variable for expression of a protein in an organism of interest, may not result in the highest possible expression level of that protein.

There is thus a need for methods that take more variables into account that potentially influence protein expression levels in an organism of interest.

### 2. BRIEF DESCRIPTION OF THE INVENTION

The invention provides a method of generating a randomized library for a gene encoding a protein of interest, comprising the steps of A) providing two or more degenerate double stranded nucleotides each comprising a fragment of 30-500 base pairs of a nucleotide molecule, whereby said double stranded fragments comprise unique single stranded overhangs of 2-10 single stranded nucleotides at either end, such that the 3' sequence of a fragment is reverse complementary to the 5' sequence of a subsequent fragment; and B) assembling said double stranded nucleotides to constitute randomized nucleotide molecules of the gene of interest. Said method further optionally comprises amplifying the assembled randomized nucleotide molecules of the gene of interest.

The invention provides a method of generating a codon-randomized library encoding a protein of interest, comprising the steps of A) providing two or more double stranded nucleotides, each comprising a fragment of 30-500 base pairs of a nucleotide molecule, each of said fragments comprising degenerate codons for a part of the protein of interest, whereby said double stranded fragments comprise unique single stranded overhangs of 2-10 single stranded nucleotides at either end, such that the 3' sequence of a fragment is reverse complementary to the 5' sequence of a subsequent fragment; and B) assembling said double stranded nucleotides to constitute codon-randomized nucleotide molecules encoding the protein of interest. Said method further optionally comprises amplifying the assembled codon-randomized nucleotide molecules encoding the protein of interest.

A method of the invention may comprise altering an amino acid residue at one or more positions in the protein of interest.

A method of the invention preferably further comprises the step of C) constructing a vector comprising the codon-randomized nucleotide molecules encoding the protein of interest. The methods of the invention will be useful in the production of many different proteins in the industrial, agricultural, chemical and pharmaceutical fields, particularly for example antibodies, hormones and other protein therapeutics.

In a preferred method of the invention, said codon-randomized, double stranded nucleotides are produced from degenerated, single stranded oligonucleotides using a primer and a DNA polymerase, preferably a strand displacing DNA polymerase. Said degenerated, single stranded oligonucleotides preferably are each 40-300 nucleotides in length, such as 50-200 nucleotides.

In a preferred method of the invention, the plurality of double stranded nucleotides is generated by a method comprising i) analyzing a nucleotide sequence encoding a protein of interest for the positioning of two or more fragments of 30 to 500 nucleotides in length, such that unique nucleotide sequences are positioned at the ends of the two or more fragments, whereby the unique nucleotide sequences are present at both facing ends of two adjacent fragments in such a way that upon assembly of the fragments the unique nucleotide sequences are part of the complete nucleotide sequence encoding the protein of interest; ii) providing codon-randomized, single stranded nucleotides for each of the two or more fragments that result from the analysis in i), said fragments comprising unique adapter sequences of 8-30 nucleotides at their 5' and 3' ends, whereby said unique adapter nucleotide sequences comprise 2-10 nucleotides from the nucleotide sequences that are positioned at the ends of the fragments of the plurality of fragments and a recognition sequence for a type IIS restriction enzyme, and whereby the type IIS restriction enzyme cuts within the unique nucleotide sequences that are positioned at the ends of the fragments of the plurality of fragments; iii) converting the single stranded polynucleotide fragments into double stranded oligonucleotides with a primer that is complementary to the adapter nucleotide sequence at the 3' end of each fragment, and a DNA polymerase; iv) optionally amplifying each of the double stranded nucleotide fragments with primers that are complementary to the adapter nucleotide sequences at the 5' and 3' ends of each fragment; and v) restricting the double stranded nucleotide fragments with the type IIS restriction enzyme to generate at either end unique overhangs of 2-10 single stranded nucleotides such that the 3' sequence of a fragment is reverse complementary to the 5' sequence of a subsequent fragment; in order to generate a plurality of double stranded nucleotides.

Said DNA polymerase preferably is a strand displacing DNA polymerase.

In a preferred method of the invention, said unique nucleotide sequences at either end comprise 4 nucleotides as single stranded overhang. Said 4 single stranded nucleotides preferably are selected from GCCC, CCAA, GGTA, ACGG, CTTA, AGTC, ACAC, ATGA, GCGA, CATA, CTGC, AACG, CGCC, AGTG, CCTC, CAGA, ACCA, AAGT, CAAC, ACGA, AGGC, AATG, GTAA, CCGA, GAGA, AGTA, TAGA, AAGG, ATAG, ACAT, ACTG, AAAT, CGAA, GACA, AGGA, ATCC, CTGA, GAAA and AGAG, preferably selected from ACGA, AGGC, AATG, GTAA, CCGA, GAGA, AGTA, TAGA, AAGG, ATAG, ACAT, ACTG, AAAT, CGAA, GACA, AGGA, ATCC, CTGA, GAAA and AGAG.

In a method of the invention, the step of assembling said double stranded nucleotides is preferably performed by ligation.

A method of the invention preferably further comprises purification of the assembled product, or of the amplified product.

A method of the invention may further comprises expressing a randomized library encoding the protein of interest in a host cell, and selecting an individual cell comprising an increased expression level of the protein of interest, when compared to other cells expressing the protein of interest. Said expression of a protein of interest in a host cell is preferably coupled to a detectable marker such as a fluorescent moiety or an antibiotic resistance moiety.

Said host cell preferably is a bacterial cell or a eukaryotic cell such as a yeast cell or a mammalian cell.

In a preferred method of the invention, the two or more double stranded nucleotide fragments of 30-500 base pairs encoding parts of a protein of interest, comprising unique overhangs at either end of 2-10 single stranded nucleotides such that the 3' sequence of a fragment is reverse complementary to the 5' sequence of a subsequent fragment, are selected by an algorithm.

The invention further provides a method of producing a protein of interest in a host cell, comprising identifying a codon-optimized sequence for a protein of interest according to the invention, and expressing said a codon-optimized sequence for a protein of interest in a host cell to produce the protein of interest.

### 3. BRIEF DESCRIPTION OF THE FIGURES

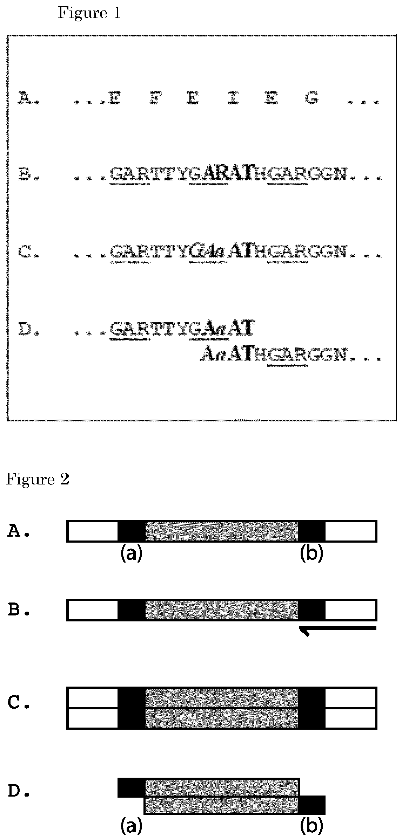

Figure 1. Design. An amino acid sequence (A) is converted to an IUPAC nucleotide notation (B, Table 1) to represent a codon degenerate sequence. The sequence is split into fragments in such a way that the ends are defined by a unique 4 nucleotide region. For this it might be necessary to fix a degenerate codon (C, italic). Ideally, this is at a position where fixation of a particular codon results in a minimal loss of codon variation. Each 4-nucleotide region should be unique within the total DNA sequence. The overhang can be used for assembly in the subsequent steps (D).
Figure 2. Fragment processing. Predefined adapters (A, white boxes) are attached upstream and downstream of the ±100 nucleotide fragment (A, gray box). The adapters contain a recognition sequence for a type IIS restriction enzyme. A primer is used on the adapter (B) to convert the ssDNA to dsDNA using PCR (C). The dsDNA is cleaned and quantified. Next, the dsDNA is cut with the correct type IIS restriction enzyme to generate dsDNA with sticky overhangs (D). Overhangs are unique as indicated by the small letter a and b between brackets.

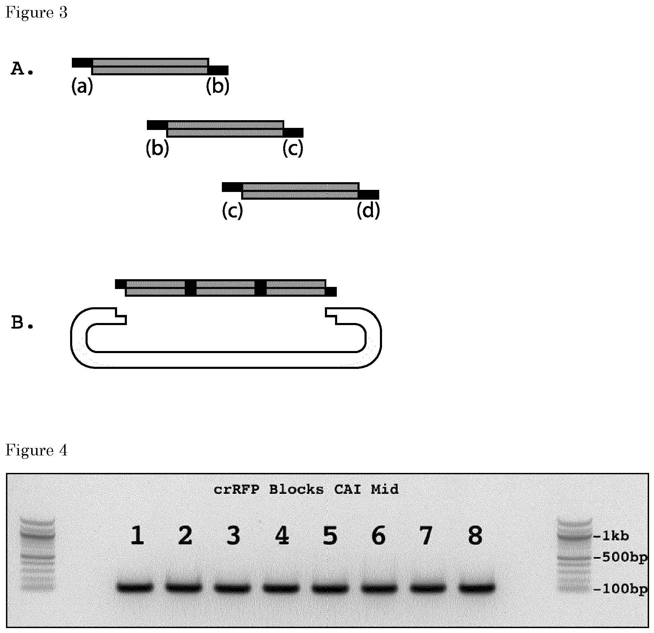

Figure 3. Assembly. Individual digested dsDNA blocks are assembled with a ligase reaction (A). Based on the unique 4 base pair overhangs, as indicated by the small letters between brackets a, b, c and d, the individual dsDNA blocks containing the codon degenerate sequence can be assembled into the full open reading frame. The assembled open reading frame is inserted into a backbone (B). The obtained plasmids are then transformed into cells.
Figure 4. 1% agarose gel showing the dsDNA blocks. The numbers identify the block as also described in Table 2.

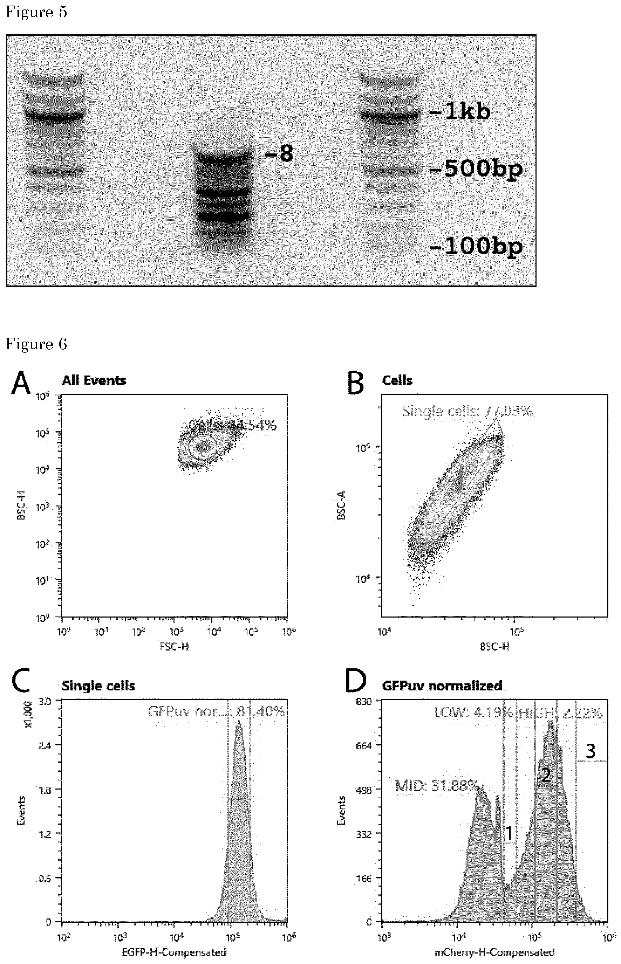

Figure 5. 1% agarose gel showing the results of the overnight assembly of all dsDNA blocks. On the outer sides a DNA ladder is shown (100 bp ladder NEB, N3231). The middle shows the results of the assembly. The "8" indicates the band where all 8 blocks are ligated together, which is the full codon randomized RFP and the band that was extracted for downstream applications. The 7 bands below are intermediate products.

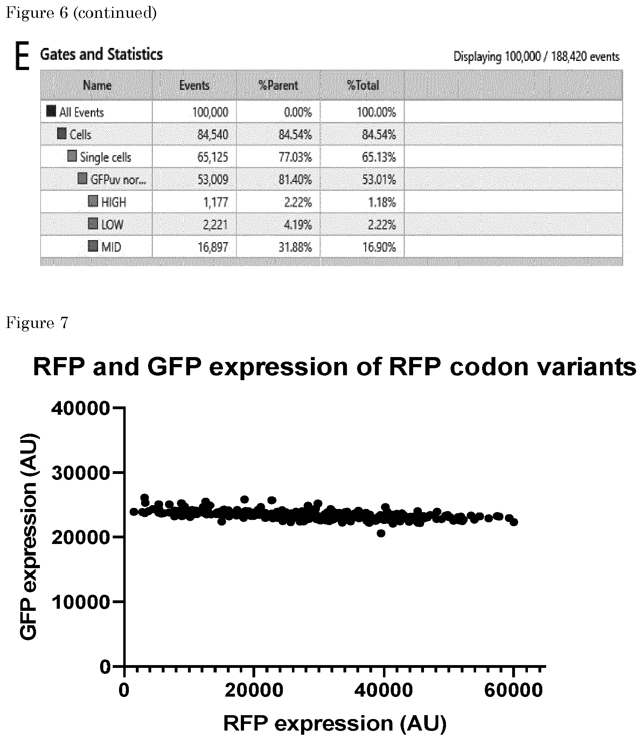

Figure 6. FACS workflow. (A) selection of cells based on size using forwards scatter and side scatter. (B) Excluding clumped cells or shapes that do not conform to standard by looking at the side scatter signal area and height. Single cells have a linear relation. (C) Limiting biological expression variance by excluding 20 % of the cells that deviate from the mean GFP expression. GFP expression is expected to be constant. (D) Sorting the cells based on RFP expression. From the right peak (these cells express RFP) the lower, mid and upper section (LOW, MID, HIGH) are sorted into separate groups for downstream applications. This sorting also excludes the left peak which are cells that have a frame shift in their RFP gene originating from the synthesis of the ssDNA by IDT as the coupling efficiency is not 100%. (E) The gates and statistics of the selection process, whereby 1 denotes low expression; 2 denotes medium expression; and 3 denotes high expression.
Figure 7. RFP and GFP expression in arbitrary units. There is a widespread in expression of RFP due to the codon randomization and as expected there was little variation in the GFP expression.

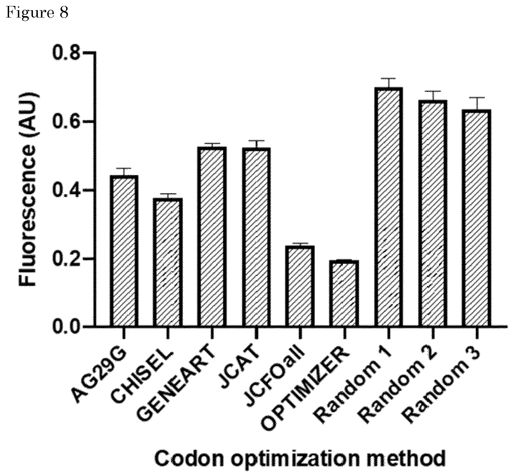

Figure 8. Benchmark results of the 3 highest expressing codon variants (random 1-3) against popular codon optimization algorithms. AG29G and JCFOall (Boël et al., 2016. Nature 529: 358-363). CHISEL (Zulkower and Rosser, 2020. Bioinformatics 36: 4508-4509). GENEART (Raab et al., 2010. Systems Synthetic Biol 4: 215-225). JCAT (Grote et al., 2005. Nucleic Acids Res 33: W526-W531). OPTIMIZER (Puigbo et al., 2007. Nucleic Acids Res 35: W126-W131). Random 1, Random 2 and Random 3 are sequence ID # 319, 318 and 317 respectively (see Table 5)

### 4. DETAILED DESCRIPTION OF THE INVENTION

### 4.1 Definitions

The term "host cell", as is used herein, refers to a prokaryotic cell or a eukaryotic cell such as a yeast cell or a mammalian cell.

The term "prokaryote" as is used herein, refers to a cellular organism that lacks an envelope-enclosed nucleus. Cellular organisms with a nucleus enclosed within a nuclear envelope are referred to as eukaryotes. Prokaryotes can be split up into two domains: Bacteria and Archaea. Bacteria comprise relatively simple genomes compared to eukaryotes. Most often, the bacterial genome consists of a single circular chromosome or a single linear chromosome, which makes bacteria ideal candidates for gene manipulation due to being a relatively simple vehicle. Preferably, *Escherichia coli* is used as prokaryote. *E. coli* is a Gram-negative, facultative anaerobic, rod-shaped, coliform bacterium of the genus *Escherichia* that is commonly found in the lower intestine of warm-blooded organisms (endotherms). Other prokaryotic organisms that can be used include Gram-positive and Gram-negative bacterial species such as species of the genus *Bacillus,* for example *Bacillus subtilis,* species of the *Clostridium* genus such as *C. acetobutilicum, C. autoethanogenum, C. beijerinckii, C. tyrobutyricum* and *C. saccharoperbutylacetonicum,* species of the genus *Streptomyces* such as *S. nodosus, S. hygroscopicus, S. avermitilis, S. verticillus, S. hygroscopicus, S. griseus, S. clavuligerus, S. venezuelae,* and *S. viridochromogenes,* species of the genus *Staphylococcus,* for example *Staphylococcus aureus,* species of the genus *Corynebacterium,* for example *C. glutamicum,* species of the genus *Pseudomonas,* for example *P. fluorescens, P. putida* and *P. auruginosa,* species of the *Amycolatopsis* genus such as *A. coloradensis, A. mediterranei,* and *A. orientalis,* species of the genus *Lactococcus,* for example *L. lactis,* and cyanobacteria such as *Arthrospira platensis.*

Further suitable prokaryotic organisms include Archaea bacteria such as for example, extremophile archaea, which are resistant either to heat or to extremes of acidity and alkalinity, including *Pyrococcus* species such as *P. furiosus, Sulfolobus species, Thermofilum* species, *Thermococcus* species such as *T. coalescens, Thermoproteus* species, *Pyrobaculum* species, *Haloquadratum* species, *Thermoplasma* species and *Ferroplasma* species. A protein that is produced in an extremophile archaea tends to be very stable, for example in organic solvents, allowing their use in environmentally friendly processes in green chemistry.

The term "eukaryotic cell", as is used herein, refers to a fungal, plant, or animal cell.

The term "fungal cell", as is used herein, refers to an organism selected from algae, including green algae such as *Chlorophyta* and *Charophyta* species such as *Neochloris oleoabundans, Chlorella* species including *Chlorella vulgaris, C. ellipsoidea, C. sorokiniana, C. kessleri,* and *C. saccharophila, Scenedesmus obliquus, Volvox carteri,* and *Chlamydomonas reinhardtii,* red algae such as *Glaucophyta* species, diatoms such as *Phaeodactylum tricornutum, Cyclotella cryptica* and *Navicula saprophila,* brown algae, golden algae, yellow-green algae, and algae of the genus *Nannochloropsis* such as *N. oculate,* a yeast such as *Saccharomyces* species such as *S. cerevisiae,* a *Kluyveromyces* species such as *K*. *lactis,* and a methylotrophic yeast such as *Komagataella phaffii* (syn. *Pichia pastoris), Hansenula polymorpha, Candida boidinii* and *Pichia methanolica,* a filamentous fungus such as an *Aspergillus* species such as *A. oryzae* and *A. niger, Mucor indicus,* a *Trichoderma* species such as *T. reesei,* and a *Rhizopus* species such as *R. oryzae.*

The term "plant cell", as is used herein, refers to a cell from an organism selected from tobacco, a cereal such as maize, rice and wheat, and a legume such as pea. Said cell may be present in a plant. A plant cell can be used for either nuclear expression or chloroplast expression, as is known to a person skilled in the art.

The term "animal cell", as is used herein, refers to an insect cell such as *Drosophila* Schneider cells, *Spodoptera frugiperda* cells Sf-9 cells and *Trichoplusia ni* cells such as High Five^{™} cells, Chinese Hamster Ovary cells (CHO), human embryonic kidney (HEK) cells and derivatives thereof including HEK293 cells including HEK293T, HEK293E, HEK-293F and HEK-293FT (Creative Biolabs, NY, USA), PER.C6^{®} cells (Thermo Fisher Scientific, MA, USA), or an animal such as *Bombyx mori* larvae or pupae.

The term "expression cassette" as is used herein, refers to a genetic construct that enables expression of a gene of interest, or genes of interest, in a cell such as a prokaryotic cell. Said expression cassette comprises elements that drive expression of a gene of interest such as a promoter sequence, optionally a 5' UTR containing a ribosome binding site, a 3' untranslated region and, optionally, a 'post stop-codon, ante terminator' (PSAT) region, and a terminator sequence. Said expression cassette preferably is present in a vector.

The term "5' untranslated region (UTR)", as is used herein, refers to the nucleotide sequence between transcription start site and the initiation codon (start codon) of a gene of interest. A 5' UTR may typically be between 30 and 500 nucleotides long. A prokaryotic 5' UTR preferably comprises a Shine-Dalgarno (SD) sequence, which is a ribosomal binding site (RBS), generally located between 3 and 10 nucleotides upstream of the start codon, which normally is AUG. The RBS is responsible for the recruitment of a ribosome during the initiation of protein translation.

The term "amplification", as is used herein, for example, refers to the increase in the number of copies of a particular DNA fragment through replication using a least one primer and a DNA polymerase. Known amplification methods include polymerase chain reaction (PCR) and isothermal amplification including, for example, helicase-dependent amplification (HDA) (Vincent et al., 2004. EMBO Rep 5: 795-800), loop-mediated amplification (LAMP) (Notomi et al., 2000. Nucleic Acids Res 28: E63), nucleic acid sequences-based amplification (NASBA) (Guatelli et al., 1990. Proc Natl Acad Sci USA 87: 1874-1878), rolling circle amplification (Ali et al., 2014. Chem Soc Rev 43: 3324-3341), strand-displacement amplification (SDA) (Walker et al., 1992. Nucleic Acids Res 20: 1691-6) and recombinase polymerase amplification (RPA) (Piepenburg et al., 2006. PLoS Biology 4: e204).

The term "vector" as is used herein, refers to a piece of DNA, either single or double stranded, such as a plasmid, a bacteriophage, or a phagemid (also termed phasmid). The vector can be for example, a plasmid, a viral genome or part thereof, or a combination thereof. The vector is used to transduce a gene encoding a protein of interest into a suitable host cell. Once in the host cell, the vector may replicate independently of, or coincidental with, the host chromosomal DNA. Alternatively, the vector can target insertion of at least an expression cassette comprising the transgene DNA into the host chromosome.

The term "coding sequence", as is used herein, refers to a portion of a gene's DNA or RNA that encodes a protein.

The term "3' untranslated region (UTR)", as is used herein, refers to the nucleotide sequence in between a stop codon of a gene of interest and a terminator of a prokaryotic expression cassette. Said 3' UTR preferably has a certain length in order to allow ribosomes to reach the stop codon at the end of the protein sequence, and not to be hindered by a secondary structure of the terminator. Said 3' UTR may be 10-1000 nucleotides, preferably 20-100 nucleotides, preferably about 25-50 nucleotides such as about 30 nucleotides.

The term "terminator", as is used herein, refers to a sequence at the end of a prokaryotic gene that defines the end of a transcriptional unit. Prokaryotic termination mechanisms are either Rho-dependent or Rho-independent. Rho factor is a helicase which assists RNA polymerase in the termination of the transcript. A most commonly used terminator is a Rho-independent terminator. Rho-independent termination relies on the formation of a GC-rich hairpin in the RNA transcript followed by a single stranded poly-uracil tract. The tertiary structure of the hairpin-DNA complex is thought to destabilize the transcription complex, initiating release of a transcribing RNA polymerase. If required, multiple terminators may be used, such as double terminators, or triple terminators, to reduce unwanted transcription and translation of downstream elements.

The term "Rho-factor", as is used herein, includes reference to Rho-factor like proteins such as homologs and analogues of the Rho protein of Escherichia coli.

The term "gene", as is used herein, refers to a stretch of nucleic acid sequences that encode one or more expression products such as proteins. On a genomic level, the term gene includes reference to all nucleic acid elements that enable expression of the one or more expression products, including promoter/enhancer sequences, coding sequences, intron sequences if present, 5' and 3' untranslated sequences, and terminator sequences. On a transcript level, the term gene includes reference to nucleic acid elements such as coding sequences and 5' and 3' untranslated sequences.

The term "promoter", as is used herein, refers to a nucleotide sequence at the 5' end of a gene onto which the transcription initiation machinery, including a RNA polymerase, binds and initiates transcription, Said promoter is often located 5-100 bp upstream of a start codon of gene. Promoters are often associated with regulatory elements, i.e. binding sites for potentially transcription activators and/or transcriptional repressors. Although promoters may vary among genomes, in eubacteria a -10 consensus sequence relative to the transcription start site (TSS), called the -10 (in E. coli: TATAAT) region and a -35 region (in E. coli TTGACA), are often present in prokaryotic promoters. The A-T-rich, -10 region is thought to facilitate unwinding of the DNA template. *Archeabacteria* often have a TATA box, or binding site for a TATA-like binding protein, at about -25 bases of a transcription start site, and a binding site for a Transcription Factor B (TFB) at about -30 to -35 of a transcription start site. Eukaryotic promoters are extremely diverse, but often contain a "TATA box" within 50 bases upstream from the transcriptional start site, and a "CAAT box" at about 70-80 bp upstream from the transcription start site.

A promoter may be constitutively active, inducible, or repressible. In addition, the strength of a promoter may range from very low to very high.

A prokaryotic promoter sequence that can be used is the Amp (*bla*) promoter. It is a weak constitutive promoter for example used for expression of ampicillin resistance gene in *E. coli.* This promoter was originally found in transposon Tn2660 upstream of the initiation codon of the structural beta-lactamase (*bla*) gene (Chen and Clowes, 1984. Nucl Acids Res. 12, 3219-3234). Other commonly used *E. coli* promoters are the tac and trc promoters. Tac and trc are controllable promoters and are hybrid promoters derived from the lac and trp promoters (Brosius, 1984. Gene 27, 151-160.). The tac promoter consists of the -35 region of the trp promoter and the -10 region of the lac promoter. The trc promoter was created by insertion of 1 base pair into the 16 base pair sequence between the consensus -10 and -35 sequences of the tac promoter, in order to obtain an optimal consensus distance of 17 base pairs between the -35 and -10 sequences. They are stronger promoters than either lac and trp because their sequences are more like the consensus sequence. Just as with lac, both the trc and tac promoters are inducible by lactose and isopropyl-β-D-thiogalactoside (IPTG). Vectors that use one of these promoters may also carry the lacO operator and the lacI gene, which encodes the repressor. Other commonly used E. coli promoter sequences are the bacterial phage T3, T7 and SP6 promoters, which are to be used in combination with the respective, host-encoded, phage RNA polymerases, either as such or in combination with regulatory elements including for instance a lac operator, a promoter of the arabinose operon, a promoter of the rhamnose operon, a promoter of the tryptophan operon, a promoter of the lac operon, a bacteriophage lambda promoter, as is known to a person skilled in the art.

Promoters for other prokaryotes are also well known, such as the Pveg, P aprE, P amyE , and P P43 promoter for B. subtilis (Kim et al., 2008. Biotechnol Bioprocess Engineering 13: 313). For *Lactobacillus,* examples of promoters include the slpA promoter and the orfX promoter (Sørvig et al., 2005. Microbiol 151: 2439-2449). Algorithms are available that may help to predict promoter regions in other prokaryotes (de Avila e Silva et al., 2011. J Theor Biol 287: 92-99; de Jong et al., 2012. BMC Genomics 13: 299). As is known to the person skilled in the art, promoters of known genes can be used in genetic manipulation (Guiziou S. et al., 2016. Nucl Acids Res 44: 7495-7508) to generate altered promoters such as stronger promoters, or promoters that also function in other prokaryotes.

Eukaryotic promoter sequences include a viral promoter such as a cytomegalovirus (CMV) promoter/enhancer, a SV40 promoter or a Rous sarcoma virus (RSV) promoter, a Cauliflower Mosaic Virus CaMV35S promoter, an alcohol oxidase 1 gene promoter (pAOX1), a formate dehydrogenase (FMD) promoter, GAL), a phosphate inducible PHO5 promoter, a copper inducible CUP1 promoter

The term "gene expression", as is used herein, refers to the process by which information from a gene is used in the synthesis of a functional protein. The process of gene expression comprises multiple steps and can be modulated at many levels. The mechanism of gene expression comprises transcription, translation, and possible post-translational modification.

The term "modulating", as is used herein in the context of protein expression, refers to regulating or adjusting a level of gene expression to a certain measure or proportion. Modulating expression of a single gene, either increasing or decreasing expression level of a gene, may enhance the overall yield of a product, especially of a functional protein. Tuning of expression of individual genes is often required when building synthetic pathways within organisms, such as for industrial biotechnological processes. Fine tuning of individual expression levels of proteins may enhance the overall yield of a product. The term modulating refers to increasing or decreasing expression of a protein of interest.

The term "poly-linker", as is used herein, refers to short segment of DNA which contains many restriction enzyme recognition sequences. A poly-linker is also known as a multiple cloning site (MCS). The purpose of a poly-linker in a vector is to facilitate a piece of DNA to be inserted into that region without disrupting the rest of the vector. A poly-linker is present in a variety of vectors, for example in pUC vectors, e.g. pUC18 and pUC19, pSF-pA-PromMCS or pSF-OXB20-BetaGal (Sigma-Aldrich). In expression vectors, a poly-linker is often located downstream of a promoter sequence to allow insertion of a nucleotide sequence encoding a protein of interest. Expression of the protein of interest in a suitable host cell is preferably controlled by the promoter sequence.

The term "transduce" and variants thereof such as "transduction", as is used herein, refers to insertion of exogenous genetic material into a host cell. The term transducing includes the transformation of nucleic acid molecules by a nucleic acid molecule such as a plasmid. The uptake of genetic material, preferably of nucleic acid molecules such as DNA or RNA, by a host cell is either by natural or artificial means. Artificial means include the generation of competent cells to render them passively permeable to genetic material, for example by incubation in the presence of calcium chloride and application of a heat shock, and electroporation by which cells are shocked with an electric field of preferably 10-20 kV/cm.

The term "codon optimized", as is used herein, refers to the selection of codons in a sequence encoding a protein of interest for optimal expression of the protein of interest in a host cell. A single amino acid may be encoded by more than one codon. For example, arginine and leucine are each encoded by a total of 6 codons. Codon optimization, i.e. the selection of a preferred codon for a specific organism at every amino acid residue, plays a critical role, especially when proteins are expressed in a heterologous system. While codon optimization may play a role in achieving high gene expression levels, other factors such as secondary structure of the messenger RNA also need to be considered. Codon optimization is offered by commercial institutions, such as ThermoFisher Scientific, called Invitrogen GeneArt Gene Synthesis, GenScript, called GenSmart^{™} Codon Optimization, or GENEWIZ, called GENEWIZ's codon optimization tool.

The term "codon harmonized", as is used herein, refers to the alignment of codon usage frequencies with those of the expression host particularly within putative inter-domain segments where slower rates of translation may play a role in protein folding. Codon harmonization may be accomplished by algorithms such as provided by Angovet al., 2011 (Angovet al., 2011. In: Evans and Xu (eds) Heterologous Gene Expression in E.coli. Methods in Molecular Biology (Methods and Protocols), vol 705. Humana Press; Claassens et al., 2017. PLoS One 12: e0184355.

The term "purifying a protein", as is used herein, refers to the purification of one or more proteins, which comprises a series of processes intended to isolate one or more proteins from a complex mixture, usually cells, tissues, and/or growth medium. Various purification strategies can be followed. For example, proteins can be separated based on size, for example in a method called size exclusion chromatography. Alternatively, proteins can be purified based on charge, e.g. through ion exchange chromatography or free-flow-electrophoresis, or based on hydrophobicity (hydrophobic interaction chromatography). It is also possible to separate proteins based on molecular conformation, for example by affinity chromatography. Said purification may involve the use of a specific tag, for example at the N-terminus and/or C-terminus of the protein. After purification, the proteins may be concentrated. This can for example be carried out with lyophilization or ultrafiltration.

The term "tag" or "tagged", as is used herein, refers to the addition of an oligo- or polypeptide, for example to the amino (N) or carboxy (C) terminus of a protein. The addition of a tag allows to isolate or immobilize a protein. Commonly used tags include a poly-histidine tag such as a 6x(His) tag, a myc tag, a glutathione-S-transferase tag, a HiBiT tag (Promega, Madison, Wisconsin), a FLAG tag, a HA tag, or multimeric tags such as a triple FLAG tag (WO2001027293). A proteinase recognition sequence may be positioned in between the protein of interest and a tag allowing removal of the tag when required.

Definitions of common terms in cell biology and molecular biology, as are used herein, can be found in The Encyclopedia of Molecular Biology, published by Blackwell Science Ltd., (1994) (ISBN 0-632-02182-9); Benjamin Lewin, (2009) "Genes X", published by Jones & Bartlett Publishing, (ISBN-10: 0763766321); Kendrew et al. (eds.) (1995), "Molecular Biology and Biotechnology: a Comprehensive Desk Reference", published by VCH Publishers, Inc., (ISBN 1-56081-569-8) and Coligan et al., eds., 2009. Current Protocols in Protein Sciences Wiley Intersciences.

Unless otherwise stated, the present disclosure can be performed using standard procedures, as described, for example in Sambrook et al., (2014) Molecular Cloning: A Laboratory Manual (4 ed.), Cold Spring Harbor Laboratory Press, Cold Spring Harbor, N.Y., USA; Davis et al., (1995) Basic Methods in Molecular Biology, Elsevier Science Publishing, Inc., New York, USA; and Berger and Kimmel Eds, (1987). Methods in Enzymology: Guide to Molecular Cloning Techniques Vol.152, S. L., Academic Press Inc., San Diego, USA, which are all incorporated by reference herein in their entireties.

### 4.2 Method for providing a plurality of double stranded nucleotides

The methods of the invention allow generation of randomized nucleotide sequences of a gene of interest. Said randomized nucleotide sequences may cover a complete gene of interest, such as for example a prokaryotic gene, or one or more parts of a gene of interest. For example, Yona et al., 2018. Nat Commun 9: 1530, Cuperus et al., 2017. Genome Res 27: 2015-2024, and de Boer et al., 2020. Nat Biotechnol 38: 56-65, describe methods for generating and screening of randomized regulatory sequences. Similarly, Hollerer et al., 2020. Nat Commun 11: 3551, and Komarova et al., 2020. Microb Biotechnol 13: 1254-1261, describe methods for generating and screening of randomized transcribed, but untranslated, nucleotide sequences. The methods as described herein below are well suited for generating and assembling randomized nucleotide fragments for any part or parts of a gene of interest, including enhancer, promoter, translated and untranslated regions, or combinations thereof.

The methods for generating a codon-randomized library encoding a protein of interest start by analyzing the nucleotide sequence encoding a protein of interest for the position of fragments that are or can be separated by unique nucleotide sequences. These fragments each are preferably 30-500 base pairs, more preferably 50-400 base pairs. The unique nucleotide sequences that separate the nucleotide sequences will allow to combine the consecutive fragments after their synthesis in the correct order. Except for the unique nucleotide sequences, the fragments are non-overlapping fragments which, after their correct assembly, together constitute a randomized coding sequence for a protein of interest.

One way of such analysis comprises the steps of providing a consensus sequence that covers all synonymous codons for all amino acid positions in a protein of interest. The consensus sequence is subsequently analyzed for the presence of unique codons for the amino acids methionine and tryptophan. Each of these codons can be part of a unique nucleotide sequence that can be used to assemble the fragments after their synthesis in a correct order. If the consensus sequence comprises codons for amino acids methionine and tryptophan, these positions can be used to determine the positioning of two or more fragments such that unique nucleotide sequences are positioned at the ends of the two or more fragments, whereby the two or more fragments together constitute the complete coding sequence for the protein of interest.

A person skilled in the art will appreciate that the methods of the invention further allow to alter the amino acid sequence of a protein of interest at one or more positions, for example at 1-50 amino acid positions, such as at 2-20 amino acid positions, including about 10 amino acid positions, in a protein of interest. In addition, said one or more alterations may reside in one or more parts of the protein of interest, for example at the N-terminus and at the C-terminus of the protein of interest. Said amino acid alteration includes the inclusion of one or more specific amino acid residues at one or more positions so as to alter the amino acid residue at the one or more positions for another amino acid residue. Said amino acid alteration also includes the inclusion of a randomized amino acid residue at one or more positions so as to alter the amino acid residue at the one or more positions for any other amino acid residue.

If the consensus sequence does not comprise codons for amino acids methionine and tryptophan, or does not comprise sufficient codons for these amino acids to divide the complete coding sequence for the protein of interest in two or more fragments, preferably of 30-500 base pairs, the position of amino acid residues that are encoded by two codons, such as cysteine, aspartic acid, glutamic acid, phenylalanine, histidine, lysine, asparagine, glutamine, and tyrosine, may be determined. In order to generate unique nucleotide sequences that can be used to assemble the fragments after their synthesis in a correct order, the codons at the indicated positions will be fixed to one specific codon encoding the required amino acid residue. The selection of a specific codon will result in a not completely randomized coding sequence for the protein of interest.

A person skilled in the art will appreciate that selection of a specific codon encoding the required amino acid residue may be influenced by features such as preferred codon usage of the host cell in which the protein of interest will be expressed, the GC content, RNA stability, and potential secondary structure formation of the resulting coding sequence. Examples of available tools to analyse such features include the Vienna RNA package (Lorenz et al., 2011. Algorithms Mol Biol 6: 26) and the mFold web server (Zuker, 2003. Nucleic Acids Res 31: 3406-3415).

In addition, algorithms for codon optimization can be used to select a specific codon encoding the required amino acid residue. Such algorithms are offered by commercial institutions, such as ThermoFisher Scientific, called Invitrogen GeneArt Gene Synthesis, GenScript, called GenSmart^{™} Codon Optimization, or GENEWIZ, called GENEWIZ's codon optimization tool.

Said unique single stranded nucleotide sequences at both ends of the fragments may comprise 1 nucleotide as a single stranded overhang, 2 nucleotides as a single stranded overhang, 3 nucleotides as a single stranded overhang, 4 nucleotides as a single stranded overhang, 5 nucleotides as a single stranded overhang, 6 nucleotides as a single stranded overhang, up to 10 nucleotides as a single stranded overhang, or a mixture thereof, provided that each single stranded overhang is used only once as a unique nucleotide sequence at the ends of two consecutive fragments.

Preferred unique single stranded nucleotide sequences at both ends of the fragments comprise 4 single stranded nucleotides, as detailed in Potapov et al., 2018 (Potapov et al., 2018. ACS Synth Biol 7: 2665-2674). Said 4 single stranded nucleotides preferably are selected from GCCC, CCAA, GGTA, ACGG, CTTA, AGTC, ACAC, ATGA, GCGA, CATA, CTGC, AACG, CGCC, AGTG, CCTC, CAGA, ACCA, AAGT, CAAC, ACGA, AGGC, AATG, GTAA, CCGA, GAGA, AGTA, TAGA, AAGG, ATAG, ACAT, ACTG, AAAT, CGAA, GACA, AGGA, ATCC, CTGA, GAAA and AGAG, preferably selected from ACGA, AGGC, AATG, GTAA, CCGA, GAGA, AGTA, TAGA, AAGG, ATAG, ACAT, ACTG, AAAT, CGAA, GACA, AGGA, ATCC, CTGA, GAAA and AGAG.

Following the positioning of the fragments and unique single stranded nucleotide sequences, the fragments may be generated. For each of the fragments, a library of single stranded DNA (ssDNA), codon randomized nucleotide sequences may be generated, for example by chemical synthesis using, for example, the phosphoramidite method of Marvin H. Caruthers (Caruthers, 1985. Science 230: 281-285).

For each of the fragments, the codon randomized nucleotide sequences preferably comprise specific adapter nucleotide sequences of 8-30 nucleotides at their 5' and 3' ends, preferably 10-25 nucleotides, more preferably 12-20 nucleotides. Said specific adapter nucleotide sequences comprise 2-10 nucleotides from the unique nucleotide sequences that are positioned at the ends of the fragments of the plurality of fragments, and a recognition sequence for a type IIS restriction enzyme. Said type IIS restriction enzyme preferably cuts within the unique nucleotide sequences that are positioned at the ends of the fragments of the plurality of fragments, such that a required unique single stranded overhang is created for each end of all fragments. Said type IIS restriction enzymes are preferably selected such that the randomized nucleotides are not cut, or only rarely cut, when said restriction enzyme is applied.

Examples of type IIS restriction enzymes include BsaI, BsmBI, BbsI, Earl, Ecil, Faul, FokI, Hgal, HphI, MboII, MlyI, Mmel, MnlI, PleI, SapI, and SfaNI. Other restriction enzymes, and their recognition sequences, are presented in databases such as REBASE (New England Biolabs), as is known in the art.

As an example, a coding sequence of a protein of interest comprises a total of about 600 nucleotides. Said coding sequence can be split into three fragments of about 200 nucleotides each. These three fragments include a first fragment encoding a N-terminal part; a second fragment encoding a middle part; and a third fragment encoding a C-terminal part, respectively.

Each of these fragments is synthesized as a single stranded nucleic acid molecule, with specific adapter nucleotide sequences of 8-30 nucleotides at their 5' and 3' ends. Said specific adapter nucleotide sequences comprise a recognition sequence for a type IIS restriction enzyme. The unique nucleotide sequences that are positioned at the ends of two adjacent fragments of the plurality of fragments are fixed. These unique nucleotides are positioned such that they will be restricted by the type IIS restriction enzyme for which a recognition sequence is present in the adapter. Following the generation of a double stranded nucleic acid molecule, and restriction with the type IIS restriction enzyme, the unique single stranded overhangs between adjacent fragments of the plurality of fragments allow assembly of the fragments in the correct order. Said type IIS restriction enzyme preferably is the same type IIS restriction enzyme for all fragments, or selected from a limited number of type IIS restriction enzymes, such as 2 or 3 type IIS restriction enzymes, for all fragments. A preferred type IIS restriction enzyme is Bsal, or a combination of Bsal and SapI.

As the unique nucleotide sequences that are positioned at the ends of two adjacent fragments of the plurality of fragments may be fixed, the methods of the invention thus may result in a slightly less than complete randomization of the coding sequence for a protein of interest, as the choice for a specific unique single stranded overhang between two successive fragments may pose some limitations, including fixation of one or more degenerate codons to a single codon.

The specific adapter nucleotide sequences at either end of each of the fragments allows the generation of double stranded fragments and, optionally, amplification of each of the fragments. It is to be understood that a particular fragment has unique adapter nucleotide sequences at each end such that, after restriction, the fragments will be assembled in their correct order. The unique adapter nucleotide sequences at the 5' end of the first fragment and at the 3' end of the final fragment need not to be digested upon assembly of the complete coding sequence. Rather, they can be used after assembly for amplification of the complete randomized coding sequence, if needed.

A person skilled in the art will understand that the methods of the invention will allow to generate randomized coding sequences as building blocks for large proteins. For example, a protein comprising 2000 amino acids will be encoded by a coding sequence of 6000 nucleotides. Rather than synthesizing the complete randomized coding sequence in fragments that will be assembled all at the same time, individual blocks of fragments can be generated and assembled prior to assembling the individual blocks into a complete randomized coding sequence. In this way, unique adapter nucleotide sequences and unique single stranded overhang sequences can be used more than once, for example to assemble two fragments in every block.

### 4.3 Methods of generating a codon-randomized library

Following the generation of codon-randomized, single stranded nucleotides for each of the two or more fragments, the single stranded polynucleotide fragments will be converted into double stranded oligonucleotides, for example with a primer that is complementary to the adapter nucleotide sequence at the 3' end of each fragment, and a DNA polymerase.

When the template is a single stranded DNA template, said DNA polymerase is a DNA dependent DNA polymerase, preferably a high fidelity DNA polymerase. Said DNA polymerase preferably has a low misincorporation rate, preferably in combination with proofreading activity. Said DNA polymerase preferably excises incorrectly incorporated mononucleotides and replaces them with the correct nucleotides to generate double stranded, codon-randomized fragments. Optionally, said two or more double stranded, codon-randomized fragments are subsequently amplified using primers that are complementary to the adapter nucleotide sequences at the 5' and 3' ends of each fragment.

Said DNA polymerase preferably is a strand displacing DNA polymerase. A strand displacing DNA polymerase refers to a polymerase that is able to displace a downstream DNA strand. Hence, said enzyme is able to unwind a double stranded DNA molecule during replication of said molecule. Because of the high level of degeneracy present in the ssDNA templates, different primers may bind to a single molecule, whereby a second primer may block elongation from a first primer by a polymerase without strand displacing activity. Examples of a strand-displacing DNA polymerase include the Klenow fragment of DNA polymerase I, a Klenow fragment of a *Bacillus stearothermophilus* DNA polymerase termed Bst polymerase (New England Biolabs, Ipswich, MA), Bsm DNA Polymerase, large fragment (Thermo Fisher Scientific, Waltham, MA), BcaBEST DNA polymerase (Takara Bio, Kusatsu, Japan), *Thermoanaerobacter thermohydrosulfuricus* DNA polymerase (US 5, 744,312), a DNA polymerase from *Thermus aquaticus, Thermus flavus* or *Thermus thermophiles,* a variant of T7 DNA-dependent DNA polymerase termed SEQUENASE (Thermo Fisher Scientific, Waltham, MA), a T5 DNA dependent DNA polymerase (Fujimura and Roop, 1976. J Biol Chem 251: 2168-2174), a T4 DNA polymerase holoenzyme (Kaboord and Benkovic, 1995. Curr Biol 5:149-157), a DNA dependent DNA polymerase from *Thermococcus litoralis,* termed VENT polymerase (New England Biolabs, Ipswich, MA), phage M2 DNA polymerase (Matsumoto et al., 1989. Gene 84: 247), phage PRD1 DNA polymerase (Jung et al., 1987. Proc Natl Aced Sci USA 84: 8287; Zhu and Ito, 1994. Biochim Biophys Acta 1219: 267-276), Phi29 DNA polymerase, and any strand-displacing DNA-dependent DNA polymerase mutant thereof including, for example, a fusion of a polymerase with a DNA binding protein (de Vega et al., 2010. PNAS 107: 16506-16511).

As an alternative, or in addition, said polymerase has 5'→3' exonuclease activity, and/or is a high fidelity DNA polymerase such as Q5^{®} High-Fidelity DNA Polymerase (NEB), and modifications thereof such as Q5U.

Said two or more double stranded, codon-randomized fragments, amplified or not, are subsequently restricted with a type IIS restriction enzyme to generate unique overhangs at either end of the fragments. Each of said overhangs preferably is 2-10 single stranded nucleotides. The fragments, and the resulting overhangs, are chosen such that the 3' sequence of a fragment is reverse complementary to the 5' sequence of a subsequent fragment such that the fragments can be assembled in an unique order to generate completed randomized coding sequence for a protein of interest.

Assembly of the restricted two or more double stranded, codon-randomized fragments, amplified or not, may be performed by, for example, ligation. The unique, single stranded nucleotide overhangs will allow the assembly of the fragments in a correct order. Assembly of the fragments, also termed ligation of the fragments, may be accomplished through the action of an enzyme by the formation of phosphodiester bonds between the 3'-hydroxyl of one DNA terminus with the 5'-phosphoryl of another. Ligation is normally performed using T4 DNA ligase, or a mutant thereof, but alternatives such as *E. coli* DNA ligase will work as well.

Said assembled, double stranded, codon-randomized nucleotide molecules encoding a protein of interest may be amplified. For this, the unique adapter nucleotide sequences at the 5' end of the first fragment and at the 3' end of the final fragment can be used for amplification of the codon-randomized coding sequences, if needed.

Following generation of assembled, double stranded, codon-randomized nucleotide molecules encoding a protein of interest, amplified or not, a vector may be constructed comprising said codon-randomized nucleotide molecules encoding the protein of interest. For this, any cloning method may be used, including traditional cloning methods may be employed, such as restriction-ligation, or Gibson Assembly (Gibson et al., 2009. Nature Methods 6: 343-345), Golden Gate Assembly (Engler et al., 2008. PLOS ONE 3 e3647), Gateway cloning (Katzen, 2007). Expert Opin Drug Discov 2: 571-89), and Topo Cloning (ThermoFisher Scientific, Waltham, MA).

Said vector may be chosen from a plasmid, a bacteriophage, a phagemid or phasmid, and a viral vector such as a retroviral vector. Preferably, the vector is a plasmid. Suitable plasmids include pCMV, pcDNA, Ti plasmid-based vectors, pYES2, pKLAC2, p15A, CoIE1, pBR322, RSF1030 and CIoDF13. Further suitable vectors are provided, for example, under the headers "Protein purification" and "Controlled expression" at addgene.org/collections/bacterial-expression. Said vector may comprise at least one origin of replication and, optionally, a selection marker such as an antibiotic resistance gene or a sensitizer such as thymidine kinase, which renders the host cell sensitive to ganciclovir selection. Possible antibiotic resistance genes include resistance genes against penicillin or derivatives thereof, aminoglycoside, chloramphenicol, macrolides such as erythromycin, azithromycin and clarithromycin, streptothricin, tetracycline, quinolone, rifampin, sulfonamide, trimethoprim, kanamycin, phleomycin D1 such as zeocin, and streptomycin. Another selection marker type for positive selection is reversal of auxotrophy, for example by cloning hisB+ in a hisB auxotroph host cell and growing it in a medium lacking histidine.

The vector further comprises a promoter for expression of the protein of interest in a suitable host cell. Said promoter may be a constitutive promoter or an inducible promoter, and may provide low, medium or high expression levels of a gene of interest.

A molar ratio of assembled, double stranded, codon-randomized nucleotide molecule encoding a protein of interest, amplified or not, to vector is preferably between 5:1 and 1:10, more preferably 1:1-1:3, most preferably about 1:2. Following ligation, the vector may be purified, if needed.

### 4.4 Protein of interest

Said POI can be any protein that one may want to produce in a host cell. Of particular interest might be commercially interesting proteins, such as enzymes sdhABC and acn involved in the production of glutamate and lysine (Brinkrolf et al., 2010. J. Biotech 149: 173-82), cytokines such as interleukins, chemokines and growth factors (Cytokine and Protein User's Guide Book, R&D systems (2018)), alcohol acyltransferase such as Atf1, AtfA and Eat1, esterases; such as Eat1, Eht1 and Eeb1, custom recombinant protein production (Ferrer-Miralles, & Villaverde, 2013. Microb Cell Fact 12: 113) or other commercially relevant enzymes such as a lipase, a protease, a phosphatase such as an alkaline phosphatase, an amylase, a phytase, and/or a cellulase (Baltaci et al., 2017. Geomicrobiol J 34: 53-62). In addition, protein complexes, such as for example CRISP/Cas ribonuclear effector complexes (Staals et al., 2013. Mol. Cell 52: 135-145; Staals et al., 2014. Mol. Cell 56: 518-530) may be efficiently produced at the required relative amounts in a host cell using the methods of the invention. For each of said proteins, a randomized coding sequence can be generated that expresses the protein to a requested level of expression in a selected host cell.

### 4.5 Host cell

The resulting vector comprising codon-randomized sequences that encode a POI may be introduced into a suitable host cell such as a prokaryotic or eukaryotic cell. Said introduction may be performed by any method known in the art, including transformation, chemical induction, microinjection, electroporation, transduction (i.e. viral transformation), and particle bombardment (Sanford et al., 1987. J Particulate Science Technology 5: 27-37). Protocols for efficient introduction of a vector into a host cell are available, allowing a person skilled in the art to perform this step without any doubts.

As is known to a person skilled in the art, cell competency is required for prokaryotic cells to take up extracellular DNA such as a plasmid by transformation. Many prokaryotic cells are naturally competent, such as *Bacillus subtilis, Lactobacillus lactis,* and *Staphylococcus aureus* (Lorenz and Wackernagel 1994. Microbiol Rev 58: 563-602). Other prokaryotes, most notably *E. coli,* may not be naturally competent and may be rendered competent through various methods. Said competent cells may for example be purchased from manufacturers such as ThermoFisher Scientific, Sigma Aldrich or New England BioLabs, or non-competent cells may be made competent with techniques such as calcium chloride treatment. Protocols are known to the person skilled in the art, and can for example be found on at international.neb.com/protocols/0001/01/01/electroporation-protocol-c2986 or at mybiosource.com/learn/testing-procedures/cacl2-transformation-technique/. Possible competent cells that are commercially available include DH10B, DH5a, and BL21(DE3) (E. coli), and all prokaryotes that are natural competent, such as *Bacillus subtilis, Lactobacillus lactis,* and

*Staphylococcus aureus.*

Subsequently, the expression levels of the POI for each synonymous coding variant are determined, preferably under the same expression conditions. In some instances, it may be possible to directly determine functional expression, e.g. as with GFP or by enzymatic action of the POI to generate a detectable optical signal. However in some instances it may be chosen to determine physical expression, e.g. by antibody probing, and rely on separate test to verify that physical expression is accompanied by the required function.

Said POI preferably is detectable by a high-throughput screening method, for example, relying on detection of an optical signal. For this purpose, it may be necessary for the POI to incorporate a tag, or be labelled with a removeable tag, which permits detection of expression. Such a tag may be, for example, a fluorescence reporter molecule translationally-fused or coupled to the C-terminal end of the POI, e.g. GFP, Yellow Fluorescent Protein (YFP), Red Fluorescent Protein (RFP) or Cyan Fluorescent Protein (CFP). Said tag may be an enzyme which can be used to generate an optical signal or a growth advantage such as an antibiotic resistance marker (e.g. TARSyn; Rennig et al., 2018. ACS Synth Biol 7: 432-442). Tags used for detection of expression may also be antigen peptide tags.

A tag may be provided for affinity purification, e.g. a His tag. Where the POI is a protein to be used as a therapeutic, any tag employed for detection of expression preferably is cleavable from the POI.

A sequence that is selected may provide the highest or optimal observed functional expression of the POI in a host cell.

POIs may preferably be recovered from the cell culture medium as secreted proteins or from host cell lysates. Said recovery may involve purification of the

POI. Purification of the POI may comprise a series of processes intended to isolate one or more proteins from a complex mixture, usually cells, tissues, and/or growth medium. Various purification strategies can be followed. For example, proteins can be separated based on size, for example in a method called size exclusion chromatography. Alternatively, proteins can be purified based on charge, e.g. through ion exchange chromatography or free-flow-electrophoresis, or based on hydrophobicity (hydrophobic interaction chromatography). It is also possible to separate proteins based on molecular conformation, for example by affinity chromatography. Said purification may involve the use of a specific tag, for example at the N-terminus and/or C-terminus of the protein. After purification, the proteins may be concentrated. This can for example be carried out with lyophilization or ultrafiltration.

### EXAMPLES

### Example 1

### Codon Randomized Gene (crGENE) Generation

The amino acid sequence of the monomeric Red Fluorescent Protein (mRFP) was used to generate an IUPAC annotated DNA sequence (Figure 1; Table 1). The IUPAC annotated sequence was then split into equal size fragments in such a way that the ends contain unique 4 base pair overhangs for later assembly of the synthesized DNA (see Figure 2). 5' and 3' flanking sequences were added to the IUPAC DNA parts which contain recognition sites for Type IIs restriction enzymes. In this example, BsaI sites were used to generate unique overhangs between adjacent fragments and SapI sites were used at the beginning and end of the gene. At the 3' flanking site additional nucleotides were added to generate a binding site for a specific primer with a melting temperature of ±54 °C. This primer also overlapped with the 4 base pair overhangs of the individual part. This primer allows for the generation of dsDNA containing degenerate codons. These dsDNA blocks were then digested to create unique 4 base pair overhangs that allow the assembly of the individual dsDNA blocks into the full open reading frame. Here, the mRFP gene was split into 8 roughly equal sized blocks. The adapter sequences, containing BsaI sites at junctions between blocks and SapI sites at the beginning and end of the open reading frame (5' of block 1 and 3' of block 8), were added to each block resulting in the final block sequences as depicted in Table 2. The ssDNA sequences were ordered at IDT (Ultramer DNA oligos). The 3' primers were ordered at IDT (Custom DNA oligos). Using the strand displacing Taq polymerase (NEB, M0482), the ssDNA was converted to double stranded DNA via PCR (Table 3). PCR reactions containing the dsDNA block were cleaned and concentrated to 20 µl mQ using the DNA Clean & Concentrator^{™}-5 kit by Zymo (D4004). 4µl Gel Loading Dye Purple (6x) (NEB, B7024) was added to each block and they were loaded on a 1% agarose gel and ran for 30 minutes at 100 volt. The dsDNA blocks are excised from the gel and purified to 20 µl mQ using the Zymoclean^{™} Gel DNA Recovery Kit by Zymo (D4002). 5 µl of the dsDNA was used to quantify the DNA concentration with the Qubit assay (Invitrogen, Q32853) according to the manufactures protocol.

The dsDNA blocks were mixed in an equal molar ratio to a total volume of 41 µl, with 5 µl T4 Ligase Buffer (NEB, B0202) 1 µl T4 Ligase (NEB, M0202) and 3 µl BsaI-HF^{®}v2 (NEB, R3733). Assembly reaction was performed at 37 °C for 18 hours, followed by 5 minutes at 60 °C and a holding step of 12 °C. The assembly was cleaned and concentrated to 15 µl mQ using the DNA Clean & Concentrator^{™}-5 kit by Zymo (D4004). 3µl Gel Loading Dye Purple (6x) (NEB, B7024) was added and the assembly mixture was loaded on a 1% agarose gel and ran for 30 minutes at 100 volt. The fully assembled product was excised from the gel and purified to 44 µl mQ using the Zymoclean^{™} Gel DNA Recovery Kit by Zymo (D4002). 1µl of SapI (NEB,) was added with 5 µl CutSmart Buffer (NEB,) and digested for 2 hours at 37 °C. The digested codon randomized mRFP was cleaned and concentrated to 15 µl mQ using the DNA Clean & Concentrator^{™}-5 kit by Zymo (D4004). The codon random RFP gene library with sticky overhangs was inserted into an expression vector driven by the bla promoter and a bicistronic design (Nieuwkoop et al., 2019. Microbial Biotechnology 12: 173-179). The vector further contained a p15A origin, a kanamycin resistance gene and GFP as an internal reference.

**Table 1 - IUPAC annotation for each amino acid. Arginine (R), Leucine (L) and Serine (S) are amino acids that can be encoded by 6 codons and can therefore not be annotated in a single IUPAC annotation. Either the first or second annotation must be used. In this example the first annotation was used as this contains the highest number of combinations. For the stop codon a single codon should be picked. In this example TAA was used.**

| **Amino acid** | **IUPAC annotation** | **Combinations** |
|---|---|---|
| A | GCN | 4 |
| R | CGN / AGR | 4 / 2 |
| N | AAY | 2 |
| D | GAY | 2 |
| C | TGY | 2 |
| Q | CAR | 2 |
| E | GAR | 2 |
| G | GGN | 4 |
| H | CAY | 2 |
| I | ATH | 3 |
| L | CTN / TTR | 4 / 2 |
| K | AAR | 2 |
| M | ATG | 1 |
| F | TTY | 2 |
| P | CCN | 4 |
| s | TCN / AGY | 4 /2 |
| T | ACN | 4 |
| W | TGG | 1 |
| Y | TAY | 2 |
| V | GTN | 4 |
| * | TAA / TGA / TAG | 1 / 1 / 1 |

### TRANSFORMATION AND SELECTION

The plasmid containing the codon randomized RFP gene library was transformed into MAX Efficiency^{™} DH10B Competent Cells (ThermoFisher Scientific, 18297010) (1µL DNA and 20 µl competent cells, 2mm cuvette, Voltage: 2500V, Resistor: 200Ω, Capacitor 25µF, BTX^{®} ECM630). Cells were recovered in 1mL NEB^{®} 10-beta/Stable Outgrowth Medium (NEB, B9035) at 37 °C for 1 hour. The cells were transferred to a 50 mL tube and 9 mL LB (10 g/L Peptone (OXOID, LP0037), 10 g/L NaCl (ACROS, 207790010), 5 g/L Yeast Extract (BD, 211929)) was added with 50 µg/mL kanamycin (ACROS, 450810500) and incubated at 37 °C overnight at 200 rpm.

A FACS (Sony, SH800S Cell Sorter) was used to sort 50.000 cells of the overnight cell culture into 3 groups based on the expression level of RFP. The left and right tail of the normal distribution and a part of the middle peak was sorted to create 3 groups of low, medium and high expression. The 3 cell groups were put on individual agar plates (10 g/L Peptone (OXOID, LP0037), 10 g/L NaCl (ACROS, 207790010), 5 g/L Yeast Extract (BD, 211929), 15 g/L Agar (OXOID, LP0011), 50 µg/mL kanamycin (ACROS, 450810500)) and grown overnight at 37 °C. From these plates individual colonies were picked and grown in 2 mL 96 well plates with 200 µL LB with kanamycin (10 g/L Peptone (OXOID, LP0037), 10 g/L NaCl (ACROS, 207790010), 5 g/L Yeast Extract (BD, 211929), 50 µg/mL kanamycin (ACROS, 450810500)) for 18 hours at 37 °C.

### MEASUREMENT AND SEQUENCING

The cell cultures were diluted 100x in PBS (8 g/L NaCl (ACROS, 207790010), 200 mg/L KCl (ACROS, 196770010), 144 mg/L Na2HPO4 (ACROS, 12499010), 240 mg/L KH2PO4 (ACROS, 447670010). RFP expression was measured using a flow cytometer (Thermo, Attune NxT Flow Cytometer). A gate was used to exclude GFP outliers (±10% of total population) and thus consequently biological variance was reduced as the GFP expression level are expected to stay constant. From the overnight cultures 1 µl of cells was used in a PCR reaction to obtain the DNA for Sanger sequencing using Q5 (NEB, M0492) (Table 4). The PCR reaction was cleaned and sequenced (Macrogen Europe B.V., Amsterdam, the Netherlands).

### RESULTS

The ssDNA and primers (Table 2) were used to generate the dsDNA (Figure 4). The dsDNA was extracted and quantified using Qubit. DNA concentration of each block was used to mix the blocks in equal ratios for a total volume of 41 µl, which was used in a 50 µl assembly reaction (see Table 4). The fully assembled codon randomized RFP was extracted from gel (Figure 5, indicated with "8"). The codon randomized RFP was inserted into an expression vector and transformed into *E.coli* cells (Colony forming units: ±320.000). After overnight growth the cells were sorted based on their GFP (an internal standard in the plasmid) and RFP (varying expression based on the codon usage of the gene) using FACS (Figure 6). From each sorted group colonies were picked and grown overnight. The cultures were measured and sequenced to obtain the codon sequence identity and associated RFP expression level. The raw sequence data was filtered to only have full read coverage and the translated codon sequence needed to translate to the RFP protein to exclude mutations that influenced the amino acid identity. A total of 319 sequences were obtained, of which three are shown in Table 5. The expression levels cover a wide range of expression (Figure 7). When compared to codon optimization algorithms, three of our best performing sequences obtained via the randomization approach outperformed all results predicted by these algorithms (Figure 8).

**Table 2 - ssDNA sequence for each generated block for mRFP. Additionally, the 3' binding primers are given for each block that were used to convert the ssDNA blocks into dsDNA via PCR.**

| Block | ssDNA Sequence (5'->3') | 3' binding primer (5'->3') |
|---|---|---|
| Block 1 | CATTCAGCTCTTCCATGGCNTCNTCNGARGAYGTNA THAARGARTTYATGCGNTTYAARGTNCGNATGGARG GNTCNGTNAAYGGNCAYGARTTYGAAATGGAGACCC GCACT | AGTGCGGGT CTCCATTTC |
| Block 2 | ACAGCGTGGTCTCGAAATHGARGGNGARGGNGARGG NCGNCCNTAYGARGGNACNCARACNGCNAARCTNAA RGTNACNAARGGNGGNCCNCTNCCNTTCGCCGGAGA CCATAGATC | GATCTATGG TCTCCGGCG |
| Block 3 | TAATAGGTCTCGCGCCTGGGAYATHCTNTCNCCNCA RTTYCARTAYGGNTCNAARGCNTAYGTNAARCAYCC NGCNGAYATHCCNGAYTAYCTGAAAGGAGACCGACG G | CCGTCGGTC TCCTTTCAG |
| Block 4 | ACTGGTCTCGGAAACTNTCNTTYCCNGARGGNTTYA ARTGGGARCGNGTNATGAAYTTYGARGAYGGNGGNG TNGTNACNGTNACNCARGAYTCNTCNCTGCGGAGAC CATCTACG | CGTAGATGG TCTCCGCAG |
| Block 5 | GCTAATTGGTCTCGCTGCARGAYGGNGARTTYATHT AYAARGTNAARCTNCGNGGNACNAAYTTYCCNTCNG AYGGNCCNGTNATGCARAARAARACCAGGAGACCCT TCAGT | ACTGAAGGG TCTCCTGGT |
| Block 6 | GAATAGGTCTCGACCATGGGNTGGGARGCNTCNACN GARCGNATGTAYCCNGARGAYGGNGCNCTNAARGGN GARATHAARATGCGNCTNAARCTNAARGACGGGGAG ACCAGTACT | AGTACTGGT CTCCCCGTC |
| Block 7 | TCAATCATGGTCTCGACGGNGGNCAYTAYGAYGCNG ARGTNAARACNACNTAYATGGCNAARAARCCNGTNC ARCTNCCNGGNGCNTAYAARACNGACATAGGAGACC GTGCGG | CCGCACGGT CTCCTATG |
| Block 8 | TGCAAGGGTCTCGCATAAARCTNGAYATHACNTCNC AYAAYGARGAYTAYACNATHGTNGARCARTAYGARC GNGCNGARGGNCGNCAYTCNACNGGNGCNTGACGAA GAGCCGTGAAT | ATTCACGGC TCTTCGTCA |

**Table 3. OneTaq PCR setup for a 50 µl reaction.**

| **Reaction setup** | | |
|---|---|---|
| **Component** | **Volume (µl)** | |
| 3' Primer (100 µM) | 2 | |
| ssDNA (100 µM) | 1 | |
| OneTaq 2X Master Mix with Standard Buffer | 25 | |
| Nuclease-free water | 22 | |
| | | |

| **Thermocycling conditions** | | |
|---|---|---|
| **Repeats** | **Temperature (°C)** | **Time (s)** |
| 1x | 94 | 30 |
| 100x | 58 | 15 |
| | 68 | 15 |
| | 12 | Hold |

**Table 4. Q5 PCR setup for transcript amplification.**

| **Reaction setup** | | |
|---|---|---|
| **Component** | **Volume (µl)** | |
| Q5 2x Master Mix | 12.5 | |
| Primer F (AATTCCGACGTCGAATTC) | 1.25 | |
| Primer R (GCGTCAGATTTCGTGATG) | 1.25 | |
| mQ | 9 | |
| Cells (O.N. culture) | 1 | |

| **Thermocycling conditions** | | |
|---|---|---|
| Repeats | **Temperature (°C)** | **Time (s)** |
| 1x | 98 | 300 |
| 35x | 98 | 10 |
| | 60 | 30 |
| | 72 | 60 |
| 1x | 72 | 120 |
| | 12 | Hold |

**Table 5. Selected sequences**

| | |
|---|---|
| 317 | ATGGCATCATCCGAAGACGTCATCAAAGAGTTCATGCGCTTCAAAGTCCGCATGGAAGGCTCTGTAAACGGCCACGAATTCGAAATTGAGGGTGAGG GTGAAGGACGTCCTTATGAGGGTACTCAAACAGCTAAACTAAAGGTTACTAAAGGTGGCCCACTTCCGTTCGCCTGGGATATCCTTTCACCTCAGTT TCAATATGGGTCTAAGGCATATGTGAAACATCCCGCAGACATACCAGACTACCTGAAACTATCGTTTCCCGAGGGGTTTAAGTGGGAGCGAGTCATG AACTTTGAAGACGGAGGGGTAGTGACAGTAACACAAGACTCGTCTCTGCAGGACGGGGAATTCATATACAAAGTCAAGCTCCGGGGCACTAATTTCC CCTCGGACGGGCCAGTCATGCAGAAAAAAACCATGGGTTGGGAAGCTTCTACTGAACGCATGTATCCTGAGGACGGCGCTCTTAAGGGGGAGATTAA GATGCGTCTGAAGCTTAAGGACGGAGGGCATTATGATGCCGAAGTAAAAACTACTTATATGGCGAAGAAGCCAGTGCAACTTCCTGGTGCATATAAA ACGGACATAAAGCTTGATATCACATCGCACAACGAAGACTACACCATCGTCGAGCAATACGAACGCGCTGAAGGCCGGCATTCAACCGGTGCCTGA |
| 318 | ATGGCATCATCTGAAGACGTCATCAAAGAATTCATGCGCTTTAAAGTCCGCATGGAAGGATCAGTTAACGGGCATGAATTCGAAATCGAGGGGGAGG GCGAAGGTCGTCCCTATGAGGGAACACAAACAGCAAAGCTCAAGGTCACAAAGGGAGGGCCGCTACCATTCGCCTGGGATATCCTCTCCCCGCAGTT CCAGTACGGCTCCAAGGCGTACGTGAAGCACCCCGCGGACATCCCTGATTATCTGAAACTCTCCTTTCCGGAGGGCTTTAAATGGGAGCGAGTGATG AATTTCGAAGATGGGGGGGTAGTAACTGTTACTCAGGACTCGTCACTGCAGGATGGAGAATTTATTTACAAGGTAAAACTTCGGGGAACAAATTTTC CGTCGGATGGTCCAGTGATGCAGAAAAAGACCATGGGATGGGAAGCTTCCACAGAGCGCATGTACCCCGAGGATGGCGCGCTAAAGGGTGAAATTAA AATGCGACTTAAGCTTAAGGACGGCGGCCATTACGATGCCGAAGTAAAAACCACCTACATGGCCAAGAAACCTGTACAACTTCCCGGTGCTTATAAA ACGGACATAAAGCTTGATATCACGTCGCATAATGAGGATTATACTATTGTGGAGCAGTATGAACGGGCCGAGGGTCGCCATTCTACTGGTGCGTGA |
| 319 | ATGGCATCATCAGAAGACGTAATTAAAGAATTTATGCGTTTTAAAGTTCGAATGGAGGGATCAGTTAACGGTCACGAATTCGAAATTGAAGGTGAGG GTGAAGGTCGTCCTTATGAGGGTACTCAGACGGCTAAGCTTAAGGTTACTAAGGGTGGTCCTCTCCCTTTCGCCTGGGATATTCTATCTCCTCAATT TCAGTATGGTTCTAAGGCCTATGTGAAGCATCCAGCCGATATTCCTGATTATCTGAAACTATCGTTTCCGGAGGGATTCAAGTGGGAGCGCGTGATG AACTTTGAAGATGGAGGCGTTGTTACAGTGACACAAGATTCATCTCTGCAGGATGGTGAGTTTATCTATAAGGTTAAGCTACGTGGGACGAATTTTC CTTCTGATGGTCCGGTTATGCAGAAGAAGACCATGGGTTGGGAGGCATCTACAGAGCGAATGTACCCGGAGGATGGTGCTCTAAAGGGCGAGATCAA GATGCGGCTGAAGCTGAAGGACGGTGGTCATTATGATGCTGAGGTGAAGACGACTTATATGGCTAAGAAGCCGGTGCAGCTGCCTGGTGCGTATAAG ACTGACATAAAACTCGACATCACCTCCCACAACGAGGACTACACCATCGTCGAGCAGTACGAACGTGCCGAGGGACGCCACTCCACCGGCGCCTGA |

## Claims

1. A method for generating a codon-randomized library encoding a protein of interest, comprising the steps of
A) providing two or more double stranded nucleotides, each comprising a fragment of 30-500 base pairs of a nucleotide molecule, each of said fragments comprising degenerate codons for a part of the protein of interest;
whereby said double stranded fragments comprise unique single stranded overhangs of 2-10 single stranded nucleotides at either end, such that the 3' sequence of a fragment is reverse complementary to the 5' sequence of a subsequent fragment;
B) assembling said double stranded nucleotides to constitute codon-randomized nucleotide molecules encoding the protein of interest and, optionally,
C) amplifying the codon-randomized nucleotide molecules encoding the protein of interest.

2. The method according to claim 1, further comprising altering an amino acid residue at one or more positions in the protein of interest.

3. The method according to claim 1 or claim 2, further comprising the step of:
D) constructing a vector comprising the codon-randomized nucleotide molecules encoding the protein of interest.

4. The method according to any one of the previous claims, wherein the codon-randomized, double stranded nucleotides are produced from degenerated, single stranded oligonucleotides using a primer and a DNA polymerase, preferably a strand displacing DNA polymerase.

5. The method according to claim 4, wherein the degenerate oligonucleotides are each 50 to 200 nucleotides in length.

6. The method according to any one of claims 1-5, wherein the plurality of double stranded nucleotides is generated by a method comprising:
i) analyzing a nucleotide sequence encoding a protein of interest for the positioning of two or more fragments of 30 to 500 nucleotides in length, such that unique nucleotide sequences are positioned at the ends of the two or more fragments;
ii) providing codon-randomized, single stranded nucleotides for each of the two or more fragments that result from the analysis in i), said fragments comprising unique adapter sequences of 8-30 nucleotides at their 5' and 3' ends, whereby said unique adapter nucleotide sequences comprise 2-10 nucleotides from the unique nucleotide sequences that are positioned at the ends of the fragments of the plurality of fragments and a recognition sequence for a type IIS restriction enzyme, and whereby the type IIS restriction enzyme cuts within the unique nucleotide sequences that are positioned at the ends of the fragments of the plurality of fragments;
iii) converting the single stranded polynucleotide fragments into double stranded oligonucleotides with a primer that is complementary to the adapter nucleotide sequence at the 3' end of each fragment, and a DNA polymerase;
iv) optionally amplifying each of the double stranded nucleotide fragments with primers that are complementary to the adapter nucleotide sequences at the 5' and 3' ends of each fragment;
v) restricting the double stranded nucleotide fragments with the type IIS restriction enzyme to generate unique overhangs at either end of 2-10 single stranded nucleotides such that the 3' sequence of a fragment is reverse complementary to the 5' sequence of a subsequent fragment; to generate a plurality of double stranded nucleotides.

7. The method according to claim 6, whereby the DNA polymerase is a strand displacing DNA polymerase.

8. The method according to any one of claims 1-7, whereby the unique nucleotide sequences at either end comprise 4 nucleotides as single stranded overhang, said 4 single stranded nucleotides preferably selected from GCCC, CCAA, GGTA, ACGG, CTTA, AGTC, ACAC, ATGA, GCGA, CATA, CTGC, AACG, CGCC, AGTG, CCTC, CAGA, ACCA, AAGT, CAAC, ACGA, AGGC, AATG, GTAA, CCGA, GAGA, AGTA, TAGA, AAGG, ATAG, ACAT, ACTG, AAAT, CGAA, GACA, AGGA, ATCC, CTGA, GAAA and AGAG, preferably selected from ACGA, AGGC, AATG, GTAA, CCGA, GAGA, AGTA, TAGA, AAGG, ATAG, ACAT, ACTG, AAAT, CGAA, GACA, AGGA, ATCC, CTGA, GAAA and AGAG.

9. The method according to any one of the previous claims, whereby the step of assembling said double stranded nucleotides is performed by ligation.

10. The method according to any one of the preceding claims, further comprising purification of the assembled product, or of the amplified product.

11. The method of identifying a codon-optimized sequence for a protein of interest, comprising expressing a randomized library encoding the protein of interest in a host cell, and selecting an individual cell comprising an increased expression level of the protein of interest, when compared to other cells expressing the protein of interest.

12. The method according to claim 11, wherein expression of the protein of interest is coupled to a detectable marker such as a fluorescent moiety or an antibiotic resistance moiety.

13. The method according to claim 11 or 12, wherein the host cell is a bacterial cell or a eukaryotic cell such as a yeast cell or a mammalian cell.

14. A method of producing a protein of interest in a host cell, comprising:
identifying a codon-optimized sequence for a protein of interest according to the method of any one of claims 11-13; and
expressing said a codon-optimized sequence for a protein of interest in a host cell to produce the protein of interest.

15. A method of generating a randomized library for a gene encoding a protein of interest, comprising the steps of
A) providing two or more degenerate double stranded nucleotides, each comprising a fragment of 30-500 base pairs of a nucleotide molecule, whereby said double stranded fragments comprise unique single stranded overhangs of 2-10 single stranded nucleotides at either end, such that the 3' sequence of a fragment is reverse complementary to the 5' sequence of a subsequent fragment,
B) assembling said double stranded nucleotides to constitute randomized nucleotide molecules of the gene of interest and, optionally
C) amplifying the assembled randomized nucleotide molecules of the gene of interest.
